# EUROPEAN PATENT APPLICATION

(11) **EP 4 755 353 A1**
(43) Date of publication of application: **10.06.2026**
(21) Application number: 24909430.1
(22) Date of filing: 25.03.2024
(51) Int. Cl.: A61F 5/00

(54) **PERFORATED DIGESTIVE TRACT IMPLANT SLEEVE AND KIT**

(30) Priority: 29.12.2023 CN 202311870249
(71) Applicant: Hangzhou Tangji Medical Technology Co., Ltd., Hangzhou, Zhejiang 310052 (CN)
(72) Inventor: LI, Wenyu, Hangzhou, Zhejiang 310052 (CN)
(74) Representative: Vogelbruch, Keang
(86) International application number: PCT/CN2024/083453
(87) International publication number: WO 2025/138460

(57) **Abstract**

The present application relates to the field of medical instruments, and provides a perforated digestive tract implant sleeve and a kit. The length of the sleeve is 60-150 cm. The sleeve comprises a proximal end and a distal end. The sleeve is provided with no more than 7 leakage holes at a distance of 20-140 cm from the proximal end and at least 10 cm from the distal end. The diameter of each leakage hole is 2-20 mm. The sleeve can achieve the isolation of chyme while allowing a very small portion of the chyme to be in contact with an intestinal wall, thereby delaying the onset of compensation function, and greatly improving the therapeutic effect on obesity and type 2 diabetes.

## Description

### CROSS REFERENCE TO RELATED APPLICATION

The present disclosure claims the priority to the Chinese patent application with the filing No. 2023118702493 filed with the Chinese Patent Office on December 29, 2023, and entitled "PERFORATED DIGESTIVE TRACT IMPLANT SLEEVE AND KIT", which is incorporated herein by reference in entirety.

### TECHNICAL FIELD

The present invention relates to the field of medical devices, in particular to an apertured digestive tract implantation sleeve and a kit (i.e., a perforated digestive tract implant sleeve and a kit).

### BACKGROUND ART

Currently, the global annual expenditure on obesity has reached an astounding 10 trillion RMB, accounting for approximately 2.8% of the global GDP. In the United States, the cost of treating obesity-related diseases accounts for nearly 17% of all medical expenses. According to obesity data published by *The Lancet* in 2021, the overweight rate among adults over 18 years old is 34.3%, and the obesity rate is 16.4%, totaling 50.7%. There are over 200 million obese individuals in China and over 700 million obese individuals worldwide. The number of obese patients is increasing year by year, which has become a global public health problem threatening human health. Therefore, there is an urgent need for a medical technology to solve the above problems.

Current treatment methods mainly rely on lifestyle interventions, medications, or surgical metabolic procedures, but these methods have certain limitations in practical application, such as low compliance, significant trauma, and irreversible damage to physiological structures. In recent years, the duodenojejunal bypass sleeve (DJBS) technique has attracted considerable interest among researchers. Based on the principle of Roux-en-Y gastric bypass (RYGB, one type of surgical metabolic procedure), DJBS involves the minimally invasive endoscopic placement of a sleeve in the duodenum and the upper segment of the jejunum. This redirects food from its previous path through the stomach and duodenum into the small intestine, to instead enter from the stomach into the distal jejunum where it mixes with bile and pancreatic juice. This isolates chyme in the duodenum and proximal jejunum from contact with the intestinal wall, thereby reducing absorption.

Regarding the approach of placing a sleeve in the proximal duodenum and jejunum, although absorption is reduced in the short term, over time, the jejunal end alters its own function and gradually undergoes compensation, thereby affecting the efficacy in terms of weight and metabolic changes. Based on this, the present invention is hereby proposed.

### SUMMARY

The objectives of the present invention include, for example, providing an apertured digestive tract implantation sleeve and a kit designed to address at least one of the problems mentioned in the background art.

Embodiments of the present invention can be implemented as follows.

In a first aspect, the present invention provides an apertured digestive tract implantation sleeve, wherein the sleeve has a length of 60-150 cm, includes a proximal end and a distal end, and is provided with one or more leakage apertures located 20-140 cm from the proximal end and at least 10 cm from the distal end, wherein the number of the leakage apertures is no more than 7, and each leakage aperture has a size of 2-20 mm.

In an optional embodiment, when a plurality of leakage apertures are provided, the leakage apertures are distributed in a paired or an unpaired configuration, wherein
the paired configuration refers to a distribution in which all the leakage apertures are divided into a plurality of pairs, each pair including at least two leakage apertures located along the same circumference, and each pair of leakage apertures is distributed along the longitudinal direction of the sleeve.

In an optional embodiment, all the leakage apertures belonging to the same pair are symmetrically or asymmetrically distributed along the same circumference.

In an optional embodiment, when the leakage apertures are distributed in the unpaired configuration, a spacing between adjacent leakage apertures in the longitudinal direction of the sleeve is 5-300 mm.

In an optional embodiment, the number of leakage apertures is 1 to 6.

In an optional embodiment, the total area of all the leakage apertures is less than or equal to 200 mm².

In an optional embodiment, each of the leakage apertures has a regular shape or an irregular shape; and
the regular shape includes a circle, an ellipse, a waist shape, or a polygon.

In an optional embodiment, the material of the sleeve is selected from at least one of PTFE, FEP, ePTFE, PU, LDPE, and LLDPE.

In an optional embodiment, the sleeve has a wall thickness of 0.01-0.03 mm.

In a second aspect, the present invention provides a digestive tract implantation kit, including a stent and the apertured digestive tract implantation sleeve according to any one of the aforementioned embodiments, wherein the proximal end of the sleeve is connected to the stent.

The embodiments of the present invention include the following beneficial effects.

The apertured digestive tract implantation sleeve obtained through the above design has a partial isolation segment provided with leakage apertures, enabling the isolation of chyme and also allowing a portion of chyme to contact the intestinal wall. This further stimulates the secretion of GLP-1, reduces absorption, further delays the exertion of intestinal compensatory function, and significantly improves the therapeutic effect on obesity and type 2 diabetes.

### BRIEF DESCRIPTION OF DRAWINGS

To describe the technical solutions in the embodiments of the present invention more clearly, the following briefly introduces the drawings required for describing the embodiments. It should be understood that the following drawings show only some embodiments of the present invention and therefore should not be considered as limiting the scope. For those of ordinary skill in the art, other related drawings can be obtained from these drawings without inventive efforts.
FIG. 1 is a schematic structural diagram of a digestive tract implantation kit according to an embodiment of the present invention; and
FIG. 2 is a statistical chart of experimental results from an experimental example.

Reference numerals: 100, sleeve; 101, proximal end; 102, distal end; 111, leakage aperture; 200, stent.

### DETAILED DESCRIPTION OF EMBODIMENTS

To make the objectives, technical solutions, and advantages of the embodiments of the present invention clearer, the technical solutions in the embodiments of the present invention will be described clearly and completely below with reference to the drawings in the embodiments of the present invention. Obviously, the described embodiments are a part of the embodiments of the present invention, rather than all of them. Generally, components of the embodiments of the present invention described and illustrated in the drawings herein can be arranged and designed in various different configurations.

Therefore, the following detailed description of the embodiments of the present invention provided in the drawings is not intended to limit the protection scope of the present invention, but only represents selected embodiments of the present invention. Based on the embodiments of the present invention, all other embodiments obtained by those of ordinary skill in the art without inventive efforts shall fall within the protection scope of the present invention.

It should be noted that similar reference numerals and letters indicate similar items in the following drawings. Therefore, once an item is defined in one drawing, it does not need to be further defined and explained in subsequent drawings.

In the description of the present invention, it should be noted that the orientation or positional relationships indicated by terms such as "up", "down", "in", and "out" are based on the orientation or positional relationships shown in the drawings, or are the orientation or positional relationships conventionally adopted when the product of the application is in use. These terms are used only for the convenience of describing the present invention and simplifying the description, and do not indicate or imply that the referred device or element must have a specific orientation, be constructed, or operate in a specific orientation. Therefore, they should not be construed as limiting the present invention.

Furthermore, terms such as "first" and "second" are used only for distinguishing description and should not be construed as indicating or implying relative importance.

It should be noted that unless in the absence of any conflict, features in the embodiments of the present invention can be combined with each other.

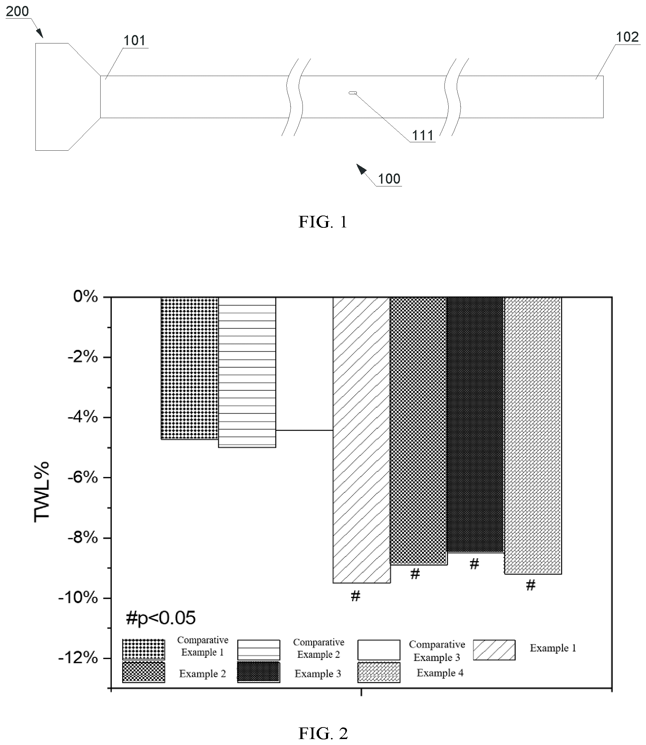

Please refer to FIG. 1. This embodiment provides an apertured digestive tract implantation sleeve 100. The sleeve 100 has a length of 60-150 cm, includes a proximal end 101 and a distal end 102, and is provided with leakage apertures 111 at least 10 cm away from the proximal end 101 and the distal end 102. The number of leakage apertures 111 is no more than 7, and each leakage aperture 111 has a size of 2-20 mm.

The apertured digestive tract implantation sleeve 100 provided by the embodiment of the present invention has a partial isolation segment provided with leakage apertures 111, enabling the isolation of chyme and also allowing a portion of chyme to contact the intestinal wall. This further stimulates the secretion of GLP-1, reduces absorption, further delays the exertion of intestinal compensatory function, and significantly improves the therapeutic effect on obesity and type 2 diabetes.

The aforementioned proximal end 101 refers to the end closer to the duodenal bulb upon implantation, and the distal end 102 refers to the end farther from the duodenal bulb.

The reason why the leakage apertures 111 along the partial isolation segment are set more than 20 cm away from the proximal end 101 is that the length of the duodenum is 20-30 cm. When a small amount of chyme leaks from the leakage apertures 111 and contacts the jejunum, it can be more conducive to the secretion of GLP-1, thereby achieving an effect similar to gastric bypass surgery (Neurogastroenterology & Motility, 2013, 25(1): e70-e79 *and* PloS one, 2013, 8(6): e65696*).* The reason for setting the aperture size of the leakage apertures 111 to 3-20 mm is that an aperture that is too small would not allow chyme to contact the intestinal tract, but an aperture that is too large would allow excessive chyme to leak out of the sleeve 100 into the intestinal tract for digestion and absorption, thereby affecting the efficacy.

Optionally, when a plurality of leakage apertures 111 are provided, the leakage apertures 111 are distributed in a paired or an unpaired configuration.

The paired configuration refers to a distribution in which all leakage apertures 111 are divided into multiple pairs, each pair including at least two leakage apertures 111 located along the same circumference, and each pair of leakage apertures 111 is distributed along the longitudinal direction of the sleeve 100.

Optionally, all leakage apertures 111 belonging to the same pair are symmetrically or asymmetrically distributed along the same circumference.

When the leakage apertures 111 are distributed in an unpaired configuration, the spacing between adjacent leakage apertures 111 in the longitudinal direction of the sleeve 100 is 5-300 mm.

Specifically, the shape of each leakage aperture 111 is regular or irregular, and the regular shape can be, for example, a circle, an ellipse, or a polygon.

When the shape of the leakage aperture 111 is circular, the aperture size refers to its diameter; and when the shape of the leakage aperture 111 is non-circular, the aperture size refers to the diameter of the circumscribed circle of the aperture.

Optionally, to achieve a better therapeutic effect on obesity and type 2 diabetes, the length of the partial isolation segment is 20-120 cm, and no more than 7 leakage apertures 111 are uniformly distributed along the partial isolation segment.

Optionally, the number of leakage apertures 111 is 1 to 6.

The total area of the leakage apertures 111 should not be too large, as an excessively large area may cause the intestinal absorption of chyme to exceed expectations. Therefore, preferably, the total area of all leakage apertures 111 is less than or equal to 200 mm².

Optionally, a material of the sleeve 100 is selected from at least one of PTFE (polytetrafluoroethylene), FEP (fluorinated ethylene propylene), ePTFE (expanded polytetrafluoroethylene), PU (polyurethane), LDPE (low-density polyethylene), and LLDPE (linear low-density polyethylene).

Optionally, to ensure the strength of the sleeve 100 while maintaining appropriate flexibility, the sleeve has a wall thickness of 0.01-0.05 mm.

An embodiment of the present invention provides a digestive tract implantation kit, including a stent 200 and the apertured digestive tract implantation sleeve 100 provided by the embodiment of the present invention, wherein the proximal end 101 of the sleeve 100 is connected to the stent 200.

Upon implantation, the stent 200 is located in the duodenal bulb, and the sleeve 100 is located in the duodenum and the upper segment of the jejunum.

### Example 1

This example was provided with a sleeve 100 having a length of 60 cm. Two leakage apertures 111 were provided at a position 40 cm from the proximal end 101. The leakage apertures 111 were distributed in pairs along the same circumference of the sleeve 100. The distance from the leakage apertures 111 to the distal end 102 of the sleeve 100 was 20 cm. The leakage apertures 111 were waist-shaped, each with a length of 15 mm and a width of 5 mm. The total area of the leakage apertures 111 was 139.25 mm².

The sleeve 100 was made of LDPE and had a wall thickness of 0.02 mm.

### Example 2

This example was provided with a sleeve 100 having a length of 100 cm. Two leakage apertures 111 in an unpaired configuration were provided on the sleeve 100. The first leakage aperture 111 was 60 cm from the proximal end 101, and the last leakage aperture 111 was 39.5 cm from the distal end 102, i.e., the spacing between the two leakage apertures 111 was 5 mm. The leakage apertures 111 were circular, each with a diameter of 10 mm. The total area of the leakage apertures 111 was 157 mm².

The sleeve 100 was made of ePTFE/FEP and had a wall thickness of 0.01 mm.

### Example 3

This example was provided with a sleeve 100 having a length of 150 cm. Six leakage apertures 111 in an unpaired configuration were provided on the sleeve 100. The six leakage apertures 111 were uniformly distributed along the longitudinal direction of the sleeve 100 at intervals of 70 mm. The first leakage aperture 111 was 80 cm from the proximal end 101. The last leakage aperture 111 was 35 cm from the distal end 102 of the sleeve 100. The leakage apertures 111 were circular, each with a diameter of 5 mm. The total area of the leakage apertures 111 was 118 mm².

The sleeve 100 was made of PU and had a wall thickness of 0.03 mm.

### Example 4

This example was provided with a sleeve 100 having a length of 80 cm. Four leakage apertures 111 distributed in two pairs were provided on the sleeve 100, each pair including two leakage apertures 111. The first pair of leakage apertures 111 was 50 cm from the proximal end 101. The last pair of leakage apertures 111 was 15 cm from the distal end 102. The spacing between the two pairs of leakage apertures 111 was 15 cm. The leakage apertures 111 within the same pair were symmetrically distributed along the same circumference. The leakage apertures 111 were rectangular, each with a length of 15 mm and a width of 3 mm. The total area of the leakage apertures 111 was 180 mm².

The sleeve 100 was made of an LDPE/LLDPE composite material and had a wall thickness of 0.05 mm.

### Comparative Example 1

This comparative example was substantially the same as Example 1, with the only difference being that no leakage apertures 111 were provided.

### Comparative Example 2

This comparative example was substantially the same as Example 1, with the only difference being that a larger number, specifically 8, leakage apertures 111 were provided.

### Comparative Example 3

This comparative example was substantially the same as Example 1 with the only difference being that the partial isolation segment was set at a position 2 cm from the proximal end.

### Experimental Example

Seventy obese patients (BMI≥ 35 kg/m²) with similar signs were divided into 7 groups, with 10 patients in each group. The sleeves provided in Examples 1 to 4 and Comparative Examples 1 to 3 were respectively implanted into the digestive tracts of the 7 groups of obese patients for a period of 3 months. The same dietary management protocol was adopted. The weight changes were recorded monthly, and the percentage of weight loss was calculated using the formula: [TWL% = (Initial weight - Weight at observation point) / Initial weight]. The results are shown in FIG. 2.

As can be seen from the results in FIG. 2, the percentage of weight loss after implantation of the sleeve 100 provided in Examples 1 to 4 of the present invention is significantly higher than that of each comparative example, indicating that providing the defined number of leakage apertures 111 at appropriate positions on the sleeve 100 is beneficial for weight reduction.

In summary, the apertured digestive tract implantation sleeve 100 provided by the embodiments of the present invention has a partial isolation segment provided with leakage apertures 111, enabling the isolation of chyme and also allowing a small amount of chyme to contact the intestinal wall. This further stimulates the secretion of GLP-1, reduces absorption, further delays the exertion of intestinal compensatory function, and significantly improves the therapeutic effect on obesity and type 2 diabetes.

The above descriptions are only specific embodiments of the present invention. However, the protection scope of the present invention is not limited thereto. Any person skilled in the art, within the technical scope disclosed in the present invention, can readily conceive of modifications or substitutions, which shall all fall within the protection scope of the present invention. Therefore, the protection scope of the present invention shall be subject to the protection scope of the claims.

### INDUSTRIAL APPLICABILITY

The apertured digestive tract implantation sleeve provided by the present invention has a partial isolation segment provided with leakage apertures, enabling the isolation of chyme and also allowing a small amount of chyme to contact the intestinal wall. This further stimulates the secretion of GLP-1, reduces absorption, further delays the exertion of intestinal compensatory function, and significantly improves the therapeutic effect on obesity and type 2 diabetes.

## Claims

1. An apertured digestive tract implantation sleeve, wherein the sleeve has a length of 60-150 cm, comprises a proximal end and a distal end, and is provided with one or more leakage apertures located 20-140 cm from the proximal end and at least 10 cm from the distal end, wherein the number of the leakage apertures is no more than 7, and each leakage aperture has a size of 2-20 mm.

2. The sleeve according to claim 1, wherein when a plurality of leakage apertures are provided, the leakage apertures are distributed in a paired or an unpaired configuration, wherein
the paired configuration refers to a distribution in which all the leakage apertures are divided into a plurality of pairs, each pair comprising at least two leakage apertures located along the same circumference, and each pair of leakage apertures is distributed along a longitudinal direction of the sleeve.

3. The sleeve according to claim 2, wherein all the leakage apertures belonging to the same pair are symmetrically or asymmetrically distributed along the same circumference.

4. The sleeve according to claim 2 or 3, wherein when the leakage apertures are distributed in the unpaired configuration, a spacing between the adjacent leakage apertures along the longitudinal direction of the sleeve is 5-300 mm.

5. The sleeve according to any one of claims 1 to 4, wherein the number of leakage apertures is 1 to 6.

6. The sleeve according to any one of claims 1 to 5, wherein a total area of all the leakage apertures is less than or equal to 200 mm².

7. The sleeve according to any one of claims 1 to 6, wherein each of the leakage apertures has a regular shape or an irregular shape; and
the regular shape comprises a circle, an ellipse, a waist shape, or a polygon.

8. The sleeve according to any one of claims 1 to 7, wherein a material of the sleeve is selected from at least one of PTFE, FEP, ePTFE, PU, LDPE, and LLDPE.

9. The sleeve according to any one of claims 1 to 8, wherein the sleeve has a wall thickness of 0.01-0.05 mm.

10. A digestive tract implantation kit, comprising a stent and the apertured digestive tract implantation sleeve according to any one of claims 1 to 9, wherein the proximal end of the sleeve is connected to the stent.
